# FASCICULE DE BREVET EUROPEEN

(11) **EP 3 941 393 B1**
(45) Date de publication et mention de la délivrance du brevet: **29.10.2025**
(21) Numéro de dépôt: 20725833.6
(22) Date de dépôt: 05.03.2020
(51) Int. Cl.: A61F 2/07, D04B 23/12, A61F 2/06

(54) **ENDOPROTHESE VASCULAIRE**
ENDOVASKULÄRE PROTHESE
ENDOVASCULAR PROSTHESIS

(30) Priorité: 18.03.2019 FR 1902757
(43) Date de publication de la demande: 26.01.2022
(73) Titulaire: MDB Texinov, 38110 Saint Didier de la Tour (FR); Deltaval, 69006 Lyon (FR)
(72) Inventeur: TANKERE, Jacques, 01800 MEXIMIEUX (FR); MIGNOT, Eric, 38620 VELANNE (FR); OTT, Franck, 38500 VOIRON (FR)
(74) Mandataire: Ipsilon
(86) Numéro de dépôt international: PCT/FR2020/050447
(87) Numéro de publication internationale: WO 2020/188180

(56) Documents cités:
- US-A1- 2006 058 862

## Description

### DOMAINE DE L'INVENTION

L'invention s'inscrit dans le domaine des endoprothèses vasculaires, plus particulièrement destinées à être mises en œuvre dans le cas d'anévrismes aortiques.

L'invention vise plus particulièrement de telles endoprothèses vasculaires intégrant une structure tubulaire textile et au moins un élément filamentaire de type alliage à mémoire de forme.

### ART ANTERIEUR

L'anévrisme est une dilatation de tronçon artériel. La rupture de cette zone pathologique est susceptible d'entraîner une hémorragie, quelque fois fatale. Lorsqu'un tel anévrisme est détecté, deux solutions sont susceptibles d'être mises en œuvre par le corps médical : la première consiste à résorber cet anévrisme par chirurgie ouverte, c'est-à-dire par une technique particulièrement lourde et invasive. La seconde consiste à shunter ledit anévrisme en insérant à son niveau une endoprothèse, implantée au sein de l'artère considérée en amont et en aval dudit anévrisme ; on parle alors de chirurgie endovasculaire. Ce shunt est destiné à permettre la circulation du flux sanguin au sein de l'endoprothèse et, corollairement, induit la diminution de la pression sanguine au sein dudit anévrisme et, par voie de conséquence, le risque de rupture de ce dernier. L'avantage de cette seconde solution est principalement de réduire de manière importante l'intervention proprement dite.

De telles endoprothèses sont classiquement constituées d'un corps textile associé à un exosquelette, typiquement réalisé en acier ou en alliage métallique, et présentant avantageusement des propriétés super élastiques ou à mémoire de forme. Cet exosquelette est usuellement suturé sur le corps réalisé en matériau textile, ou associé au stent lors d'une opération dite d'electrospinning dans le domaine considéré. Le corps en matériau textile est par exemple réalisé en PET (polyéthylène téréphtalate).

Les matériaux à mémoire de forme sont aujourd'hui largement connus et répandus. Ils présentent la caractéristique de pouvoir atteindre des niveaux de déformation relativement importants, de manière réversible. Ce phénomène est dû à une transformation de phase cristallographique au sein du matériau lorsque ce dernier est soumis à un chargement mécanique - à une contrainte - et/ou à une variation de température. Ainsi, les matériaux à mémoire de forme sont capables de développer plusieurs propriétés particulièrement intéressantes, telles que la superélasticité et la ferroelasticité (mémoire de forme). Parmi les alliages à mémoire de forme, on connaît notamment ceux à base de nickel et de titane, tels que par exemple ceux connus sous la marque Nitinol.

On a ainsi décrit, par exemple dans le documents US 2006/058862 A1 et US 6,814,747, une telle endoprothèse comprenant une enveloppe tubulaire textile, à l'extérieur de laquelle sont rapportés de manière plus ou moins périodique, des anneaux réalisés en alliage à mémoire de forme, susceptibles de s'expanser sous l'effet de la température, et en l'espèce typiquement sous l'effet de la température du corps humain, de telle sorte à conférer à l'endoprothèse la forme tubulaire requise pour assurer sa fonction de shunt, et corollairement, à conférer à l'endoprothèse une forme suffisamment réduite pour permettre son introduction dans l'artère considéré, par exemple au moyen d'un cathéter.

Si, incontestablement, ce type d'endoprothèse a permis un progrès notable dans le traitement de l'anévrisme par chirurgie endovasculaire, il présente cependant un certain nombre d'inconvénients.

Parmi ceux-ci, on peut tout d'abord citer le cas des endofuites, c'est-à-dire un défaut d'étanchéité de l'endoprothèse mise en place. Ces endofuites sont inhérentes à la structure proprement dite de la gaine tubulaire, typiquement réalisée par tissage, outre des zones de suture de l'exosquelette en alliage à mémoire de forme sur ladite gaine, susceptibles de créer des zones de faiblesse au niveau de la gaine tubulaire, et donc corollairement des orifices au travers desquels est susceptible de s'échapper le flux sanguin. Lorsqu'une telle endoprothèse est par exemple implantée au niveau de l'aorte, notamment abdominale, et lorsque l'on connaît la pression du flux sanguin à ce niveau, ces fuites peuvent rapidement devenir un problème, et notamment ne pas permettre la résorption de l'anévrisme que l'endoprothèse est justement destinée à réduire.

Une autre difficulté inhérente à l'utilisation des endoprothèses de l'art antérieur réside dans le défaut de positionnement, et un comportement insatisfaisant au niveau des plicatures, c'est-à-dire des tortuosités qu'est susceptible d'adopter l'endoprothèse pour s'adapter aux propres méandres de l'artère au niveau de laquelle est destinée à être implantée l'endoprothèse. De telles plicatures de l'endoprothèse sont susceptibles de conduire à une occlusion à tout le moins partielle de cette dernière, se traduisant par des complications importantes nécessitant une nouvelle intervention chirurgicale.

Encore une autre difficulté résultant des endoprothèses de l'art antérieur réside dans leur hétérogénéité. En effet, en raison de la suture ou du mode de fixation des bagues ou système équivalent, et de manière générale de l'exosquelette sur la structure textile, l'endoprothèse présente un comportement mécanique qui s'éloigne de celui des tissus natifs, conduisant à des rejets de l'endoprothèse plus ou moins rapides, nécessitant leur remplacement périodique en raison des nouveaux risques d'occlusion susceptible d'intervenir et, ne conduisant pas à l'effet recherché, en l'espèce la résorption de l'anévrisme. Par ailleurs, les hétérogénéités des structures endoprothétiques de l'art antérieur peuvent conduire à un glissement potentiel de ladite structure au sein de l'artère - migration -, cela menant inéluctablement à la nécessité d'une seconde opération très invasive.

### DESCRIPTION SOMMAIRE DE L'INVENTION

L'invention a pour objectif une endoprothèse vasculaire visant à s'affranchir de ces différents inconvénients.

Elle a donc pour objet une endoprothèse vasculaire constituée d'une structure textile tricotée tubulaire, intégrant au sein des mailles de ladite structure textile tricotée au moins un fil de trame continu hélicoïdal, s'étendant selon toute la dimension principale de l'endoprothèse, ledit au moins un fil réalisé en un alliage à mémoire de forme ayant préalablement subi une éducation telle, et notamment un traitement thermomécanique tel, qu'il lui confère des propriétés super-élastiques ou lors de la transformation austénitique résultant de la température du corps humain, de telle sorte à ce que ledit fil confère à la structure sa forme tubulaire.

En d'autres termes, l'invention consiste à supprimer toute forme d'exosquelette et des éventuelles sutures de ce dernier sur la structure textile de l'art antérieur, conférant à l'endoprothèse une homogénéité optimale, et développant, en raison de l'optimisation des mailles de la structure textile tricotée et du comportement thermomécanique de la trame, un comportement biomimétique le plus abouti possible.

En raison de l'intégration au sein de sa structure et plus précisément au sein des mailles de la structure textile tricotée d'un fil continu en alliage à mémoire de forme, celui-ci confère donc, une fois l'endoprothèse en place, la forme tubulaire recherchée, afin de permettre à celle-ci de remplir sa fonction première, à savoir d'autoriser la circulation en son sein du flux sanguin.

Selon l'invention, la structure textile tricotée est obtenue sur métier Rachel double fonture, ou sur métier à crochets, selon une armure Charmeuse, ou autres liages similaires du type double tricot, chainette tramée, ou toute autre armure permettant d'obtenir de bonnes caractéristiques de biomimétisme de la structure implantée, et selon les caractéristiques mécaniques recherchées, notamment l'élasticité, l'extensibilité ou autre comportement.

Il convient en outre de préciser, qu'en raison des commandes électroniques des métiers à tricoter mis en œuvre, il est possible de changer d'armure en cours de fabrication, et par exemple de disposer de zones de densité ou de caractéristiques différentes.

La structure textile en question est réalisée à base de PET (polyéthylène téréphtalate) ou de tout autre fil biocompatible polymère (polypropylène, ePTFE - polytétrafluoroethylène expansé, par exemple) et/ou résorbable ou biodégradable (par exemple de type PGA - acide polyglycolique).

Le fil de trame, réalisé en matériau à mémoire de forme et avantageusement en Ni-Ti, est inséré de manière continue au sein des mailles de ladite structure textile tricotée, l'insertion étant réalisée au niveau de la double fonture avec décalage de cette insertion dans le sens production, cette trame continue étant acheminée au niveau des deux fontures par révolution autour de ces dernières, et donc concomitamment avec la réalisation des deux nappes textiles résultant du tricotage au niveau des deux fontures. L'objectif de la trame en Ni-Ti est avant tout d'assurer les fonctions principales de l'endoprothèse, et en particulier un déploiement optimal de la structure au sein de l'anévrisme lors de la chirurgie opératoire endovasculaire.

Il peut ainsi en résulter une disposition du fil de trame en hélice. Le squelette métallique est donc directement intégré en continu au sein de la structure textile, de manière particulièrement homogène et sans aucune suture, contrairement aux endoprothèses de l'art antérieur, qui présentent quant à elles des exosquelettes globalement suturés au textile menant alors à des structures hétérogènes.

Selon l'invention, le fil de trame typiquement réalisé en alliage nickel-titane, peut subir lui-même une ondulation préalable avant son insertion au sein de la double fonture. Cette ondulation particulière du fil de trame, dont l'amplitude et le pas sont pilotés et imposés lors du traitement thermique spécifique dudit fil, permet d'apporter plus de souplesse à la structure et d'aider à la phase finale d'écrasement, mieux connu dans le domaine considéré sous l'expression anglo-saxonne « crimping » nécessaire pour permettre l'insertion de ladite structure au sein de son cathéter en vue de son implantation in situ.

Le fil de trame réalisé en alliage nickel-titane, présente un diamètre compris entre 50 et 200 micromètres. Si en effet, le diamètre du fil est inférieur à 50 micromètres, l'expérience démontre que la fonction première conférée au fil, à savoir donner à l'endoprothèse sa forme tubulaire après transformation austénitique, est insuffisante, et des risques d'occlusion sont susceptibles d'apparaître.

Si en revanche, le diamètre du fil constitutif de la trame est supérieur à 200 micromètres, l'endoprothèse devient trop rigide et son comportement s'éloigne de manière trop importante du biomimétisme recherché, susceptible de conduire à un risque de migration de l'endoprothèse au sein de l'artère dans laquelle elle est introduite et corollairement, à des risques de fuites.

Selon une autre caractéristique de l'invention, la déformation longitudinale de l'endoprothèse, c'est-à-dire selon sa dimension principale, est comprise entre 0 et 30 %. Cette déformation peut s'avérer nécessaire pour rester proche d'un comportement biomimétique et ainsi d'un écoulement optimum et homogène du flux sanguin, réduisant les risques de perturbations sur d'autres zones des artères. Elle est inhérente à la seule structure textile tricotée qui permet ces ajustements de la courbe de résistance-allongement.

Selon une caractéristique avantageuse de l'invention, l'endoprothèse une fois réalisée est soumise à une compaction de ses parois par une action mécanique, puis enduite d'un revêtement de surface spécifique (composé majoritairement de collagène, d'albumine ou de gélatine, cette solution d'enduction étant avantageusement complétée de composants nécessaires à la biocompatibilité de la structure, telles que l'héparine, le carbone ou les fluoropolymères), de telle sorte à conférer à ladite endoprothèse le niveau de perméabilité requis au regard de la viscosité du flux sanguin, et donc corollairement éviter les fuites, et typiquement une perméabilité voisine de 0,1ml/cm²/min déterminée selon la norme ISO 25539-2.

L'invention vise également un procédé pour la réalisation de cette endoprothèse vasculaire. Ce procédé consiste :
▪ à réaliser par tricotage à mailles jetées sur un métier à double fonture, deux nappes solidarisées entre elles au niveau de leurs bords respectifs sens production pour définir à terme une structure tubulaire ;
▪ et à insérer une trame continue réalisée en fil à mémoire de forme au niveau de la double fonture, venant s'insérer dans les mailles sur lesdites deux nappes avec décalage dans le sens production, la trame continue étant acheminée au niveau des fontures par révolution autour desdites fontures, typiquement de manière hélicoïdale par rapport au sens production de la structure.

Selon une caractéristique avantageuse de l'invention, le pas de l'hélice est constant et, afin d'aboutir à la compacité requise et optimiser l'étanchéité de l'endoprothèse, ce pas est de 1/1, c'est-à-dire que le fil de trame réalisé en matériau à mémoire de forme fait un tour de la structure textile tubulaire toutes les mailles. Cependant, selon les caractéristiques recherchées, ce pas peut être de 1/2 à 1/10.

### BREVE DESCRIPTION DES FIGURES

D'autres caractéristiques et avantages de l'invention ressortiront clairement de la description qui en est faite ci-après, à titre indicatif et nullement limitatif, en référence aux dessins annexés, dans lesquels :
La figure 1 est une vue schématique illustrant une endoprothèse conforme à l'invention mise en place au sein de l'aorte abdominale.
La figure 2 est une vue schématique de deux types d'endoprothèse conforme à l'invention, illustrant respectivement une endoprothèse à diamètre constant (partie gauche), et une endoprothèse munie d'un jambage obtenu directement en cours de réalisation (partie droite), les deux types d'endoprothèse pouvant être assemblés lors de l'implantation sur le patient.
La figure 3 est une vue schématique d'une endoprothèse conforme à l'invention dans sa phase de réalisation sur métier double fonture, faisant apparaître le fil de trame à propriétés super élastiques.
La figure 4 est une représentation schématique d'un détail de la figure 3.
La figure 5 est représentation schématique vue du dessus du principe mis en œuvre par le procédé de réalisation de l'endoprothèse de l'invention.
La figure 6 est une représentation schématique vue du dessus d'une double fonture avec représentation de l'insertion du fil de trame à mémoire de forme venant mailler au niveau desdites fontures.
La figure 7 est une représentation schématique en perspective d'un dispositif apte à mettre en œuvre le procédé de l'invention.

### DESCRIPTION DETAILLEE DE L'INVENTION

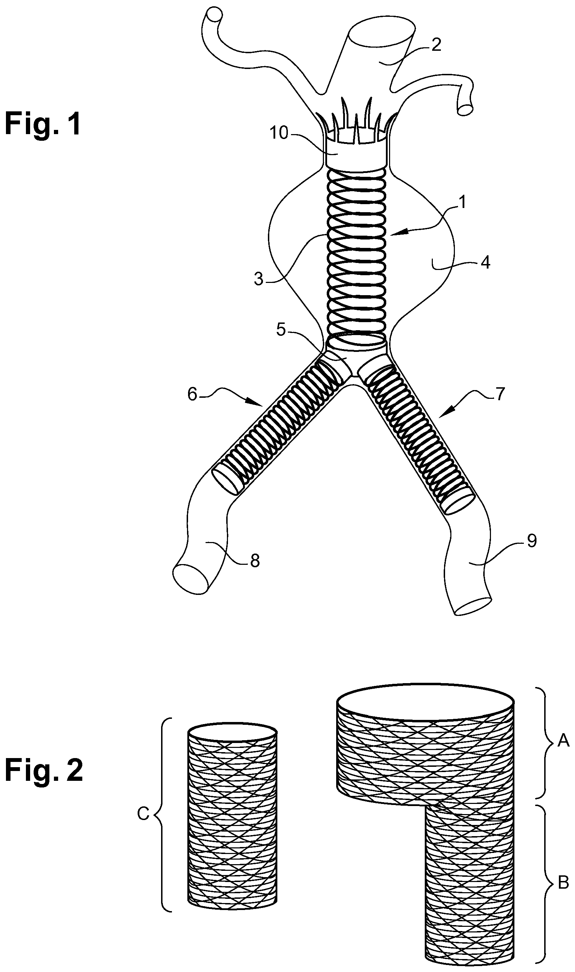

On a schématiquement décrit en relation avec la figure 1, l'implantation d'une endoprothèse (1) conforme à l'invention au sein de l'aorte abdominale (2). Pour la simplicité de l'illustration, seul le fil de trame continu (3) réalisé en alliage à mémoire de forme, et en l'espèce en alliage nickel-titane, tel que par exemple en Nitinol a été représenté. On peut bien observer son cheminement hélicoïdal, résultant du mode de réalisation de ladite prothèse, et au demeurant, décrit plus en détail ultérieurement.

Dans cet exemple, l'endoprothèse principale, destinée à shunter l'anévrisme (4), se scinde au niveau d'une zone de jonction (5) en deux endoprothèses secondaires (6, 7), destinées à être implantées dans le début des deux artères fémorales (8, 9).

La zone supérieure de l'endoprothèse est fixée au niveau de l'arc aortique. En l'occurrence, l'expérience démontre que la zone de fixation (10) aux collets aortiques doit être renforcée par rapport au corps de la structure afin d'éviter tout risque de migration une fois la structure implantée *in-vivo.* Le fil de trame en nickel-titane peut donc, dans ladite zone, présenter un diamètre ou un comportement thermomécanique différent de manière à assurer le maintien de l'endoprothèse.

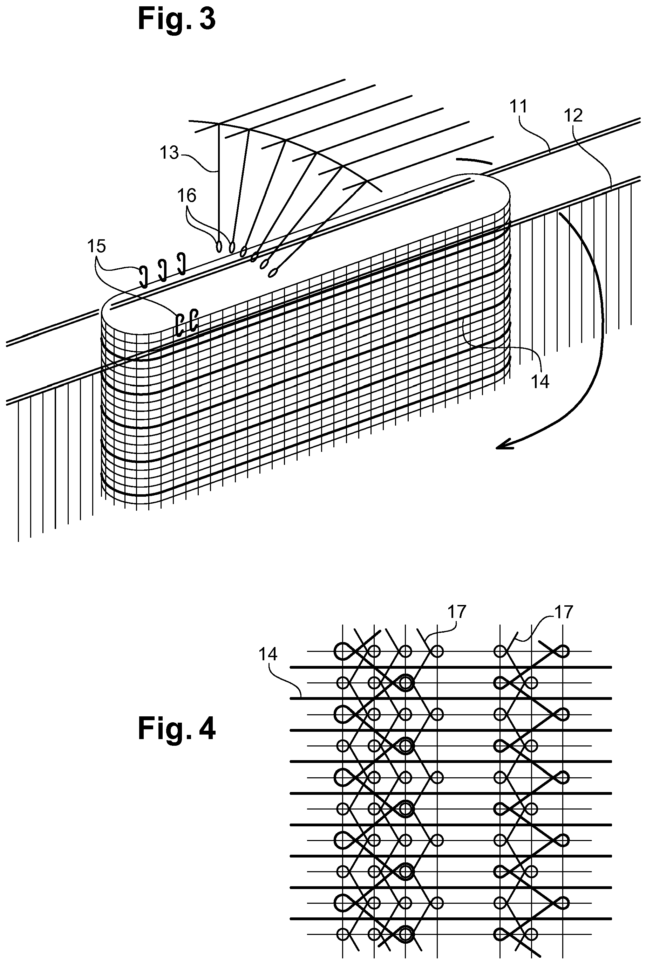

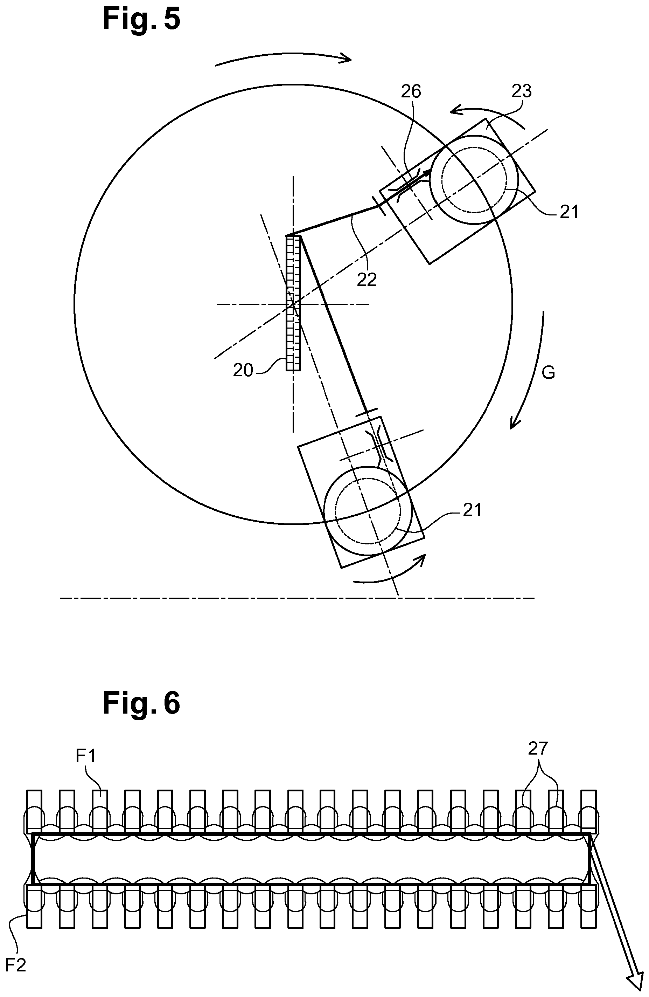

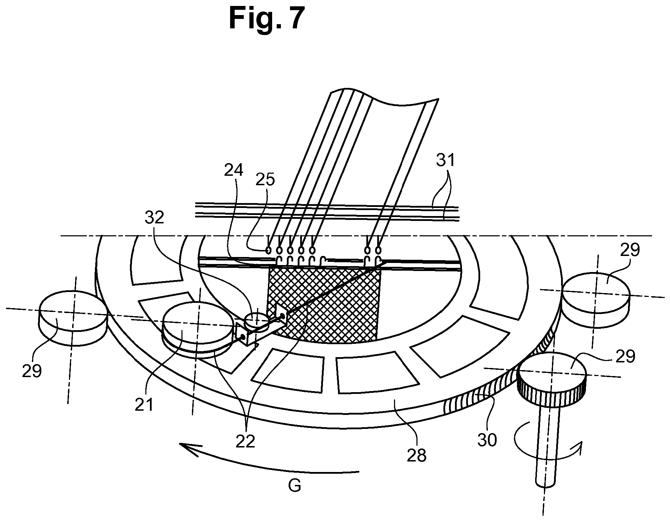

La figure 3 schématise la réalisation de l'endoprothèse conforme à l'invention sur les deux fontures (11, 12) d'un métier Rachel avec les barres de fils maillantes (13) et le fil de trame continu en alliage nickel-titane (14). Ce dernier passe alternativement sur chacune des deux fontures tout en s'intégrant dans les mailles de chaque côté (aiguilles (15) et passettes (16)) par un dispositif rotatif (voir figures 5 et 7), selon un pas de 1/1 ou de 1/2 à 1/10 par arrêt dudit dispositif rotatif. On a illustré par la flèche la rotation du fil de trame (14) autour des deux fontures.

On a représenté au sein de la figure 4 un détail de la figure 3. Plus précisément, on a schématisé le maillage au sein des fontures. Apparaissent ainsi les fils (17) constitutifs de la structure, et par exemple réalisés en PET venant mailler autour du fil de trame (14).

Ce mode de réalisation permet en outre de réduire par programmation, la largeur du tube défini à terme par la structure textile 3D au cours du cycle de fabrication de façon à réaliser l'un des jambages ou endoprothèses secondaires (6, 7) d'une prothèse d'anévrisme de l'aorte, destinées à venir s'insérer dans l'une ou les deux artères fémorales (voir figure 2).

Selon cette forme particulière de réalisation (figure 2 partie droite), le deuxième jambage (6, 7) est réalisé séparément sur le métier à tricoter, selon un diamètre inférieur à celui correspondant à l'endoprothèse principale. L'assemblage est réalisé lors de la pose sur le patient par emboitage et fixation selon un mode respectant les exigences des endoprothèses.

La zone de jonction (5) entre les différentes portions (3, 6 et 7) est réalisée par tricotage, par tissage ou tout autre moyen d'assemblage, selon une géométrie propre à la configuration souhaitée, et permet de lier l'ensemble de la structure de manière continue.

Cette endoprothèse secondaire est introduite in situ à l'aide d'un deuxième cathéter comme cela se fait usuellement pour les endoprothèses. Dans ce cas, lors de la pose, la partie plus étroite vient s'accrocher dans la première partie lors de la sortie du cathéter. Le diamètre du jambage est légèrement plus élevé que celui de la partie qui l'accueille, ce qui lui permet d'exercer un effort sur la première partie, suffisant pour garantir son maintien in vivo. Selon le besoin, des crochets ou accroches supplémentaires peuvent être ajoutés afin d'exclure tout risque de glissement ou de séparation des différentes portions.

La mise en place de cette endoprothèse in situ est réalisée à l'aide d'un ou plusieurs cathéters, au sein duquel est insérée ladite endoprothèse. Cette insertion est généralement consécutive à une étape d'écrasement, mieux connu sous l'expression anglo-saxonne « crimping » de la structure 3D constitutive de ladite endoprothèse. Une fois cette dernière en place, en l'espèce au sein de l'aorte abdominale, la super-élasticité du fil de trame en alliage nickel-titane entraine le retour (après écrasement pour permettre son insertion dans le cathéter) de la structure dans sa forme initiale, et notamment tubulaire afin de permettre à ladite endoprothèse de remplir la fonction qui lui est dévolue, et plus particulièrement permettre le passage du flux sanguin et corollairement résoudre l'anévrisme.

On a représenté en relation avec la figure 5 le principe du procédé de réalisation de l'endoprothèse de l'invention. Fondamentalement, il est généré une structure 3D à l'aide d'un métier Rachel ou métier crochet double fonture (20) (pour lequel, pour la simplification de la compréhension, on n'a pas représenté les modules d'alimentation en fils).

Autour de ce métier double fonture, tourne (flèche G) une bobine (21) d'alimentation en fil de trame (22) en alliage nickel-titane ayant auparavant subi un traitement thermique pour optimisation de son comportement thermomécanique, montée sur un support (23). De fait, le fil de trame vient donc tramer au niveau des aiguilles (24) et des passettes (25) montées sur les fontures du métier RACHEL au fur et à mesure de la réalisation de la structure 3D à ce niveau.

A cet effet, la bobine (21) d'alimentation en fil de trame est disposée dans un plan s'inscrivant perpendiculairement à celui recevant les doubles fontures et passant au niveau de la zone de coopération des aiguilles et des passettes desdites fontures.

Le mouvement rotatif de la bobine autour des deux fontures est obtenu par tout moyen, et en tout état de cause, par un mécanisme synchronisé avec le cycle de formation des mailles de la structure 3D sur le métier double fonture. Cette bobine délivre le fil de trame après passage par un dispositif de freinage (26), afin d'assurer une tension correcte du fil de trame. Ce freinage s'effectue soit directement sur le fil de trame, soit sur la bobine elle-même. De tels systèmes de freinage sont connus en soit. Ils peuvent être notamment de nature mécanique, électrique, voire électromagnétique.

La programmation du pas de l'hélice décrite par le fil de trame par rapport au sens production de la structure 3D peut être gérée directement en concomitance avec le programme de tricotage de la structure de fond 3D. Typiquement, cette programmation est telle que l'on dispose après imprégnation ou enduction d'une perméabilité voisine de 0,1ml/cm²/min, en tout cas conforme à la norme applicable pour ladite structure 3D déterminée, et notamment apte à remplir sa fonction d'étanchéité la plus appropriée pour contenir le flux sanguin appelé à transiter en son sein.

Le diamètre du tube résultant de la structure 3D est typiquement de 20 millimètres. Cependant, ce diamètre est susceptible de varier entre 5 et 40 millimètres, en fonction des patients et des zones d'application de l'endoprothèse, dont l'usage au demeurant, n'est pas limité aux seuls anévrismes aortiques, mais également dans d'autres pathologies vasculaires, notamment lorsqu'une substitution ou un renfort interne s'avère nécessaire.

Par ailleurs, on fixe le pas de l'hélice du fil de trame de façon à ce que ce que ledit fil de trame effectue un tour de la structure textile tubulaire toutes les mailles constitutives, ou toutes les 2 à 10 mailles de cette dernière (typiquement avec une densité de maille de l'ordre de 7 à 20 mailles /cm). La structure alors réalisée permet d'assurer un comportement thermomécanique optimal et d'éviter les risques de plicatures, endofuites et migrations une fois l'endoprothèse introduite au sein de la portion artérielle pathologique.

La double fonture a en outre été représentée schématiquement en vue de dessus sur la figure 6. On a ainsi matérialisé les fontures avant (F1) et arrière (F2), au niveau desquelles apparaissent les fils de tricotage (27) de la structure support 3D, ainsi que la disposition schématique du fil de trame inséré au moyen d'un dispositif, tel qu'illustré sur la figure 7.

Un tel dispositif comporte typiquement une bobine (21) d'alimentation en fil de trame (22), montée sur une couronne circulaire (28). Cette couronne circulaire est donc montée autour du métier double fonture. Elle est mue en rotation par exemple au moyen de pignons dentés (29), actionnés en rotation par des moteurs électriques (non représentés). Ces pignons dentés viennent engrener sur le bord périphérique denté (30) de ladite couronne. Un système de transmission à courroie ou tout autre moyen d'entrainement peut être utilisé pour assurer la rotation. La gestion du ou des moteur(s) électrique(s) actionnant les pignons dentés est synchronisée avec le cycle de fonctionnement du métier double fonture, de telle sorte à introduire le fil de trame au moment opportun au niveau de chacune des fontures.

Ainsi donc, le fil de trame effectue une révolution, et dans l'exemple décrit une rotation, autour des fontures dans la zone où est réalisée la structure textile 3D obtenue par le jeu des organes de tricotage montés sur des fontures, respectivement aiguilles et passettes. Les passettes sont elles-mêmes mues sur des barres support (31), selon le programme de liage des fils retenu.

On a également représenté sur cette figure le dispositif de freinage (32) positionné en sortie de la bobine, aux fins de réguler la tension du fil de trame.

Selon l'invention, et après réalisation de l'endoprothèse, on procède à une enduction plein bain de cette dernière avec une solution par exemple de collagène. Ce faisant, on optimise le niveau de perméabilité mesuré selon la norme ISO 25539-2 applicable pour l'endoprothèse, qui s'avère suffisant et respecte les exigences en vigueur. Cette enduction peut également être réalisée par d'autres techniques disponibles, telles que par exemple par pulvérisation.

On conçoit tout l'intérêt de l'endoprothèse de l'invention. En premier lieu, il convient de souligner son caractère biomimétique, résultant d'une part, de sa structure homogène et de son mode de réalisation, supprimant toute notion d'exosquelette et de suture, et d'autre part, du motif des mailles de la structure tricotée 3D. Ce faisant, on favorise la durabilité de son implantation puisque les risques de migration in-vivo sont limités, et son corollaire, la diminution des risques de rejet. En second lieu, on s'affranchit des risques d'occlusion quelle que soit la tortuosité des artères que l'endoprothèse est susceptible de rencontrer pour se conformer à l'anatomie particulière de certains vaisseaux sanguins. Enfin, on évite tout risque de fuite, notamment en raison de la structure maillée de la structure 3D.

## Revendications

1. Endoprothèse vasculaire (1) constituée d'une structure textile tricotée tubulaire, intégrant au sein des mailles de ladite structure textile tricotée au moins un fil de trame continu hélicoïdal (3, 14, 22), s'étendant selon toute la dimension principale de l'endoprothèse, ledit au moins un fil (3, 14, 22) étant réalisé en un alliage à mémoire de forme ayant préalablement subi une éducation telle, et notamment un traitement thermomécanique tel que, à la température du corps humain, ledit fil confère à la structure sa forme tubulaire par super élasticité ou effet mémoire de forme.

2. Endoprothèse vasculaire selon la revendication 1, ***caractérisée* en ce que** la structure textile tricotée est obtenue sur métier Rachel double fonture ou sur métier à crochets, selon une armure choisie dans le groupe Charmeuse, double tricot ou chainette tramée.

3. Endoprothèse vasculaire selon l'une des revendications 1 et 2, ***caractérisée* en ce que** le diamètre de la structure textile tricotée est compris entre 5 et 40 millimètres.

4. Endoprothèse vasculaire selon l'une des revendications 1 et 2, ***caractérisée* en ce qu'**elle comporte un jambage obtenu après réduction du diamètre de la structure textile tricotée, ledit jambage étant configuré pour constituer une endoprothèse secondaire (6, 7).

5. Endoprothèse vasculaire selon l'une des revendications 1 à 4, ***caractérisée* en ce que** la structure textile tricotée est réalisée en un matériau biocompatible choisi dans le groupe comprenant le PET (polyéthylène téréphtalate), le polypropylène, l'ePTFE, et les matériaux biodégradables ou biorésorbables de type PGA.

6. Endoprothèse vasculaire selon l'une des revendications 1 à 5, ***caractérisée* en ce que** le fil de trame (3, 14, 22), réalisé en matériau à mémoire de forme est en Nickel-Titane, et **en ce qu'**il est inséré de manière continue au sein des mailles de ladite structure textile tricotée.

7. Endoprothèse vasculaire selon l'une des revendications 1 à 6, ***caractérisée* en ce que** le fil de trame présente un diamètre compris entre 50 et 200 micromètres.

8. Endoprothèse vasculaire selon l'une des revendications 1 à 7, ***caractérisée* en ce que** la déformation longitudinale de l'endoprothèse, c'est-à-dire selon sa dimension principale, est comprise entre 0 et 30 %.

9. Endoprothèse vasculaire selon l'une des revendications 1 à 8, ***caractérisée* en ce qu'**elle est enduite de collagène, d'albumine ou de gélatine, de telle sorte à conférer à ladite endoprothèse une perméabilité voisine de 0,1ml/cm²/min au regard de la viscosité du flux sanguin, et donc corollairement éviter les fuites tout en respectant la biocompatibilité de la prothèse.

10. Endoprothèse vasculaire selon la revendication 9, ***caractérisée* en ce que** la solution d'enduction de collagène, d'albumine ou de gélatine comprend également de l'héparine, du carbone ou un fluoropolymère.

11. Procédé pour la réalisation d'une endoprothèse vasculaire, consistant :
▪ à réaliser par tricotage à mailles jetées sur un métier à double fonture, deux nappes solidarisées entre elles au niveau de leurs bords respectifs sens production pour définir à terme une structure tubulaire ;
▪ et à insérer une trame continue réalisée en un fil à mémoire de forme au niveau de la double fonture, venant s'insérer dans les mailles sur lesdites deux nappes avec décalage dans le sens production, la trame continue étant acheminée au niveau des fontures par révolution autour desdites fontures, typiquement de manière hélicoïdale par rapport au sens production de la structure.

12. Procédé pour la réalisation d'une endoprothèse vasculaire selon la revendication 11, ***caractérisé* en ce que** le pas de l'hélice décrite par le fil de trame à mémoire de forme est constant et de 1/1, c'est-à-dire que le fil de trame fait un tour de la structure textile tubulaire toutes les mailles.

13. Procédé pour la réalisation d'une endoprothèse vasculaire selon la revendication 11, ***caractérisé* en ce que** le pas de l'hélice décrite par le fil de trame à mémoire de forme est constant et est compris entre 1/2 et 1/10.

14. Procédé pour la réalisation d'une endoprothèse vasculaire selon l'une des revendications 11 à 13, ***caractérisée* en ce que** le métier à double fonture est un métier RACHEL ou un métier à crochets, dans lequel le fil de trame (3, 14, 22) est inséré au niveau de la double fonture dudit métier avec décalage de cette insertion dans le sens production, cette trame continue étant acheminée au niveau des deux fontures par révolution autour de ces dernières, et donc concomitamment avec la réalisation des deux nappes textiles résultant du tricotage au niveau des deux fontures.

15. Procédé pour la réalisation d'une endoprothèse vasculaire selon la revendication 14, ***caractérisée* en ce que** le fil de trame subit lui-même une ondulation préalable avant son insertion au sein de la double fonture du métier RACHEL ou du métier à crochets.

## Patentansprüche

1. Gefäßendoprothese (1), bestehend aus einer schlauchförmigen gestrickten Textilstruktur, in deren Maschen mindestens ein kontinuierlicher, spiralartiger Schussfaden (3, 14, 22) integriert ist, der sich über die gesamte Hauptabmessung der Endoprothese erstreckt, wobei der mindestens eine Faden (3, 14, 22) aus einer Formgedächtnislegierung besteht, die zuvor einer Behandlung, insbesondere einer thermomechanischen Behandlung, unterzogen wurde, sodass der Faden der Struktur bei Körpertemperatur durch Superelastizität oder Formgedächtniseffekt seine Schlauchform verleiht.

2. Gefäßendoprothese nach Anspruch 1, **dadurch *gekennzeichnet,* dass** die gestrickte Textilstruktur auf einer doppelfonturigen Rachel-Strickmaschine oder einer Häkelmaschine in einer Legung hergestellt wird, die aus der Gruppe Charmeuse, Doppelstrick oder Kettengewirk ausgewählt ist.

3. Gefäßendoprothese nach einem der Ansprüche 1 und 2, **dadurch *gekennzeichnet,* dass** der Durchmesser der gestrickten Textilstruktur zwischen 5 und 40 Millimetern liegt.

4. Gefäßendoprothese nach einem der Ansprüche 1 und 2, **dadurch *gekennzeichnet,* dass** sie ein Beinelement umfasst, das nach Verringerung des Durchmessers der gestrickten Textilstruktur erhalten wird, wobei das Beinelement so konfiguriert ist, dass es eine sekundäre Endoprothese (6, 7) bildet.

5. Gefäßendoprothese nach einem der Ansprüche 1 bis 4, **dadurch *gekennzeichnet,* dass** die gestrickte Textilstruktur aus einem biokompatiblen Material hergestellt ist, das aus der Gruppe ausgewählt ist, die PET (Polyethylenterephthalat), Polypropylen, ePTFE und biologisch abbaubare oder bioresorbierbare Materialien vom Typ PGA umfasst.

6. Gefäßendoprothese nach einem der Ansprüche 1 bis 5, **dadurch *gekennzeichnet,* dass** der aus Formgedächtnismaterial hergestellte Schussfaden (3, 14, 22) aus Nickel-Titan besteht und dass er kontinuierlich in die Maschen der gestrickten Textilstruktur eingetragen wird.

7. Gefäßendoprothese nach einem der Ansprüche 1 bis 6, **dadurch *gekennzeichnet,* dass** der Schussfaden einen Durchmesser zwischen 50 und 200 Mikrometern aufweist.

8. Gefäßendoprothese nach einem der Ansprüche 1 bis 7, **dadurch *gekennzeichnet,* dass** die Längsverformung der Endoprothese, d. h. an ihrer Hauptabmessung, zwischen 0 und 30 % liegt.

9. Gefäßendoprothese nach einem der Ansprüche 1 bis 8, **dadurch *gekennzeichnet,* dass** sie mit Kollagen, Albumin oder Gelatine beschichtet ist, um der Endoprothese eine Durchlässigkeit von etwa 0,1 ml/cm²/mind. in Bezug auf die Viskosität des Blutflusses zu verleihen und gleichzeitig Leckagen zu vermeiden, während die Biokompatibilität der Prothese gewahrt bleibt.

10. Gefäßendoprothese nach Anspruch 9, **dadurch *gekennzeichnet,* dass** die Beschichtungslösung aus Kollagen, Albumin oder Gelatine auch Heparin, Kohlenstoff oder ein Fluorpolymer enthält.

11. Verfahren zur Herstellung einer Gefäßendoprothese, bestehend aus:
▪ der Herstellung von zwei Lagen, die an ihren jeweiligen Produktionskanten miteinander verbunden sind, durch Kettenwirkverfahren auf einer doppelfonturigen Maschine, um schließlich eine schlauchförmige Struktur zu definieren;
▪ und dem Eintrag eines kontinuierlichen Schusses aus einem Formgedächtnisfaden in Höhe der doppelten Fontur, der in die Maschen auf den beiden Lagen mit Versatz in Produktionsrichtung eingetragen wird, wobei der kontinuierliche Schussfaden auf Höhe der Fonturen durch Umwicklungen der Fonturen, typischerweise spiralförmig in Bezug auf die Produktionsrichtung der Struktur, befördert wird.

12. Verfahren zur Herstellung einer Gefäßendoprothese nach Anspruch 11, **dadurch *gekennzeichnet,* dass** die Steigung der Spirale, die der Formgedächtnis-Schussfaden beschreibt, konstant und gleich 1/1 ist, d. h. der Schussfaden geht durch alle Maschen der schlauchförmigen Textilstruktur.

13. Verfahren zur Herstellung einer Gefäßendoprothese nach Anspruch 11, **dadurch *gekennzeichnet,* dass** die Steigung der Spirale, die der Formgedächtnis-Schussfaden beschreibt, konstant ist und zwischen 1/2 und 1/10 liegt.

14. Verfahren zur Herstellung einer Gefäßendoprothese nach einem der Ansprüche 11 bis 13, **dadurch *gekennzeichnet,* dass** die doppelfonturige Maschine eine RACHEL-Maschine oder eine Häkelmaschine ist, bei der der Schussfaden (3, 14, 22) an der Doppelfontur der Maschine mit einem Versatz dieses Eintrags in Produktionsrichtung eingetragen wird, wobei dieser kontinuierliche Schuss an beiden Fonturen durch Umwicklungen dieser und somit gleichzeitig mit der Herstellung der beiden Textillagen befördert wird, die durch das Stricken an den beiden Fonturen entstehen.

15. Verfahren zur Herstellung einer Gefäßendoprothese gemäß Anspruch 14, **dadurch *gekennzeichnet,* dass** der Schussfaden selbst vor seinem Eintrag in die Doppelfontur der RACHEL-Maschine oder der Häkelmaschine vorgewellt wird.

## Claims

1. A vascular stent graft (1) formed of a tubular knitted textile structure, integrating within the meshes of said knitted textile structure at least one helical continuous weft yarn (3, 14, 22), extending all along the major dimension of the stent graft, said at least one yarn (3, 14, 22) made of a shape memory alloy having previously been submitted to such an education, and particularly such a thermomechanical treatment, that, at the human body temperature, said yarn gives the structure its tubular shape by superelasticity or shape memory effect.

2. The vascular stent graft according to claim 1, ***characterized* in that** the knitted textile structure is obtained on a double needle bed Rachel loom or on a hook loom, according to a weave selected from the Charmeuse, double knit, or weft chain group.

3. The vascular stent graft according to any of claims 1 and 2, ***characterized* in that** the diameter of the knitted textile structure is in the range from 5 to 40 millimeters.

4. The vascular stent graft according to any of claims 1 and 2, ***characterized* in that** it comprises a limb obtained after reduction of the diameter of the knitted textile structure, said limb being configured to form a secondary stent graft (6, 7).

5. The vascular stent graft according to any of claims 1 to 4, ***characterized* in that** the knitted textile structure is made of a biocompatible material selected from the group comprising PET (polyethylene terephthalate), polypropylene, ePTFE, and biodegradable or bioresorbable materials of PGA type.

6. The vascular stent graft according to any of claims 1 to 5, ***characterized* in that** the weft yarn (3, 14, 22), made of a shape memory material is made of nickel-titanium, and **in that** it is continuously inserted into the meshes of said knitted textile structure.

7. The vascular stent graft according to any of claims 1 to 6, ***characterized* in that** the weft yarn has a diameter in the range from 50 to 200 micrometers.

8. The vascular stent graft according to any of claims 1 to 7, ***characterized* in that** the longitudinal deformation of the stent graft, that is, along its major dimension, is in the range from 0 to 30%.

9. The vascular stent graft according to any of claims 1 to 8, ***characterized* in that** it is coated with collagen, albumin, or gelatin, to provide said stent graft with a permeability close to 0.1 ml/cm²/min regarding the viscosity of the blood flow, and thus as a corollary to avoid leaks while respecting the biocompatibility of the stent graft.

10. The vascular stent graft according to claim 9, ***characterized* in that** the collagen, albumin, or gelatin coating also comprises heparin, carbon, or a fluoropolymer.

11. A method of forming a vascular stent graft, comprising:
▪ forming by warp stitch knitting on a double needle bed loom two layers joined together at the level of their respective edges in the production direction to eventually define a tubular structure;
▪ and inserting a continuous weft made of a shape memory yarn at the level of the double needle bed, inserting into the meshes on said two layers with an offset in the production direction, the continuous weft being conveyed to the level of the needle beds by revolution around said beds, typically helically with respect to the production direction of the structure.

12. The method of forming a vascular stent graft according to claim 11, ***characterized* in that** the pitch of the helix followed by the shape memory yarn is constant and 1/1, that is, the weft yarn makes turns around the tubular textile structure once for every stitch.

13. The method of forming a vascular stent graft according to claim 11, ***characterized* in that** the pitch of the helix followed by the shape memory yarn is constant and is in the range from 1/2 to 1/10.

14. The method of forming a vascular stent graft according to any of claims 11 to 13, ***characterized* in that** the double needle bed loom is a RACHEL loom or a hook loom, wherein the weft yarn (3, 14, 22) is inserted at the level of the double needle bed of said loom with an offset of this insertion in the production direction, this continuous weft being conveyed to the level of the two needle beds by revolution around the latter and thus concomitantly to the forming of the two textile layers resulting from the knitting at the level of the two needle beds.

15. The method of forming a vascular stent graft according to claim 14, ***characterized* in that** the weft yarn itself undergoes a previous corrugation before its insertion into the double needle bed of the RACHEL loom or of the hook loom.
